# EUROPEAN PATENT SPECIFICATION

(11) **EP 1 420 782 B1**
(45) Date of publication and mention of the grant of the patent: **13.08.2008**
(21) Application number: 01958395.4
(22) Date of filing: 22.08.2001
(51) Int. Cl.: A61K 31/4015, A61P 25/28

(54) **USE OF NEFIRACETAM FOR TREATING POST-STROKE NEURODEGENERATION**
VERWENDUNG VON NEFIRACETAM ZUR BEHANDLUNG VON POSTISCHEMISCHER NEURODEGENERATION
UTILISATION DE LA NEFIRACETAME DANS LE TRAITEMENT DES AFFECTIONS NEURODEGENERATIVES POST-ISCHEMIQUES

(43) Date of publication of application: 26.05.2004
(73) Proprietor: Hamilton Pharmaceuticals, Inc., Washington, DC 20006 (US)
(72) Inventor: OTOMO, Eiichi, Tama-shi, Tokyo 206-0013 (JP); TAKASU, Yoshiyuki, DAIICHI PHARMACEUTICAL CO., LTD, Tokyo 134-0081 (JP)
(74) Representative: Reitstötter - Kinzebach
(86) International application number: PCT/JP2001/007180
(87) International publication number: WO 2003/018005

(56) References cited:
- EP-A- 1 020 189
- EP-A- 1 022 029
- US-A- 5 886 023
- DATABASE EMBASE [Online] ELSEVIER SCIENCE PUBLISHERS, AMSTERDAM, NL; ENDERBY P. ET AL: "Effect of piracetam on recovery and rehabilitation after stroke: A double- blind, placebo-controlled study." retrieved from STN Database accession no. 94237142 XP002170792 & CLINICAL NEUROPHARMACOLOGY, (1994) 17/4 (320-331).,

## Description

### TECHNICAL FIELD

The present invention concerns the use of a cyclic gamma-aminobutyric acid (GABA) derivative, namely N-(2,6-dimethylphenyl)-2-(2-oxo-1-pyrrolidinyl)acetamide or its salt, for the preparation of medicaments for the treatment of neurodegeneration after a stroke, for improving the Activities of Daily Living (ADL) after a stroke or for recovering, or at least for improving the recovery of, a post-stroke patient.

### BACKGROUND ART

It is known that a way to assess physical impairment in post-stroke patients, besides the neurological motor and sensory examination, is to quantitate deficits in the performance of daily activity (ADL) according to assessment scales, such as the John Hopkins Functioning Inventory (JHFI) or analogous ones, which determine the patients' ability to accomplish normal operations, in particular keeping the sitting or standing position, walking, washing, dressing, undressing, taking the meals, bath and using lavatory. The assessment, the impairment, as well as and the need of recovery of ADL after stroke are illustrated by Robert G. Robinson "The Clinical Neuropsychiatry of Stroke", 1998, Cambridge University Press, pages 143, 222-225 and 292-293).

Depression is the main disorder associated with stroke and there is a correlation between the severity of depression and the severity of impairment in ADL. For the improvement in the impairment of ADL and, as a consequence, for the improvement in the recovery from stroke, patients are mainly treated with antidepressant drugs and, according to the above cited reference, there are no documented pharmacological treatments, apart from said antidepressants treatments, which improve physical or cognitive recovery from completed stroke (Robert G. Robinson, loc. cit., page 293).

Cyclic GABA derivatives, more particularly 2-oxopyrrolidine derivatives, are compounds extensively used in pharmaceutical compositions for the improvement of memory and attention and are known as mneniotonic or nootropic agents. Typical drugs of this class include 2-oxo-1-pyffolidineacetamide (piracetam), 1-(4-methoxybenzoyl)-2-pyrrolidinone (aniracetam) and 4-hydroxy-2-oxo-1-pyrrolidineacetamide (oxiracetam).

It is known (BE 883791 - US 4341790) that anilides of 2-oxo-1-pyrrolidineacetamide show central vasoactive and tranquillizing properties as well as the ability of regulating the metabolism and inhibiting thrombocyte agglutination. Thus, said compounds are deemed to be useful for the treatment of cerebro-ischaemic or atrophic diseases, brain irrigation disorders, brain atrophic crises as well as of brain aging processes. Among these anilides of 2-oxo-1-pyrrolidineacetamide, the 2,6-dirnethylanilide, i.e. N-(2,6-dimethylphenyl)-2-(2-oxo-1-pyrrolidinyl)acetamide, known and hereinafter referred to as nefiracetam, represented by the formula (A) 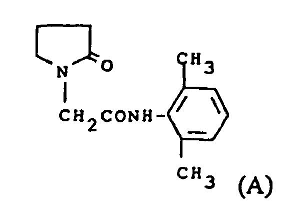 has been reported to be effective in prolonging the survival time upon a decrease in blood oxygen level and in relieving failure of memory due to cerebropathy.

The literature extensively discloses (see for example E. Ohtomo et al., J Clin. Exp. Med., Suppl., 1994, 170/9, 777-816) the usefulness of nefiracetam in improving psychiatric disorders associated with cerebrovascular diseases such as stroke (cerebral infarction or cerebral hemorrhage), this activity being a consequence of the favorable action of nefiracetam on the cerebral irrigation, as suggested by BE 883791.

It is also known (K. Hirata et al., Brain Topography 1996, 8/3, 279-284) that nefiracetam acts as a cerebral metabolic enhancer in improving the mental function impairment in stroke patients, thus confirming the suggestion of BE 833791 which disclosed the metabolism-regulating properties of the compound. Hirata et al., however, conclude that the improvement of mental function tests was not significant.

Moreover, it is known (US 5886023) that nefiracetam improves symptoms of cerebrovascular or Alzheimer's type dementia due to a decline in mental function. EP-A-1 022 029 describes the use of nefiracetam as a nootropic agent.

All these documents indicate that the efficacy of nefiracetam in the symptomatic treatment of impaired mental function is due to its ability in improving the cerebral irrigation or to its metabolism-regulating properties. Psychiatric symptoms and cognitive impairment are frequently observed following stroke and negatively affect both the patient and the caregiver.

In the above-cited article of Ohtomo et al., the global results of a clinical study, conducted in two groups of patients to which nefiracetam and, respectively, placebo were administered after a stroke (cerebral infarction or cerebral hemorrhage), showed that the compound improves the psychiatric symptoms but concluded that there was no significant difference between the two groups as far as the activities of daily living were concerned. Thus, according to these results nefiracetam appeared as inactive in improving the disturbances of the activities of daily living in post-stroke patients. A positive effect in this indication could suggest a curative use of a drug for the recovery from a stroke or, at least, for an improved recovery from stroke.

### DISCLOSURE OF INVENTION

It has now surprisingly been found that, if nefiracetam is administered to a patient, suffering from the consequences of a stroke, within the first 12 months after the stroke a significant improvement with regard to the global disturbancies of the activities of the daily living is obseved.

More particularly, it has been found that nefiracetam is able to induce an improvement in the recovery of post-stroke patients, provided that said nefiracetam is administered within the first 12 months after the stroke.

Moreover, it has surprisingly been found that nefiracetam possesses a remarkable neurotrophic activity which allows the regeneration of damaged neurons, thus being able to combat neurodegeneration, and that nefiracetam is particularly effective when the neurodegeneration is due to a stroke (cerebral infarction or cerebral hemorrhage).

Thus, it is an object of the present invention to provide the use of nefiracetam or its salt for the preparation of a medicament for the treatment of post-stroke neurodegeneration wherein said treatment is initiated more than 6 months and within 12 months after said stroke.

The mechanism by which nefiracetam acts on the neurodegeneration, thus allowing, for example, a recovery or at least an improvement in the recovery from stroke, is unknown because nefiracetam does not possess known biochemical activities which are predictive and known for this action such as, for example an agonism for the 5HT1-A receptor or a positive modulation, of AMPA-sensitive glutamate receptors (AMPA indicates α-amino-3-hydroxy-5-methyl-4-isoxazolepropionic acid).

The neurotrophic, in particular antineurodegenerative, property of nefiracetam was inferred on the basis of the clinical evidence of a significant improvement in the Activities of the Daily Living and other psychiatric symptoms by early treatment and confirmed by biochemical and animal tests.

Thus, it is another object of the present invention to provide the use of nefiracetam or its salt for the preparation of medicaments for improving the Activities of Daily Living (ADL) in a post-stroke patient as defined above. The treatment comprises administering to a patient an effective dose of nefiracetam, said administration being initiated more than 6 months and within the first 12 months after the stroke.

In order to display the best activity, nefiracetam or its salt will be administered early or at least as soon as possible within said time intervall.

Examples of nefiracetam's salts can include acid addition salts with inorganic or organic acids such as citric acid, tartaric acid, hydrochloric acid, sulfonic acid and the like.

Nefiracetam or its salt can be administered in various manner to achieve the desired effect, for example for improving ADL in a post stroke patient or for the recovery of, or at least for improving the recovery of, a post-stroke patient. The compound can be administered alone or in the form of pharmaceutical compositions comprising a phamacologically effective amount of nefiracetam as an active ingredient and a pharmaceutical acceptable carrier to the patient to be treated, preferably orally. The oral amount of nefiracetam administered will vary and can be any effective amount according to the physician's prescription. Normally, depending upon the patient and the mode of administration, the quantity of compound administered orally may vary over a wide range to provide from about 1 mg/kg to about 20 mg/kg, usually 1.5 mg/kg to 15 mg/kg of body weight of the patient per dose. Unit doses of nefiracetam in the oral pharmaceutical compositions may contain, for example, from about 50 mg to about 1200 mg, usually from 100 to 600 mg of the compound and may be administerd 1 to 4 times daily.

The activity of nefiracetam to improve ADL in post-stroke patients has been discovered during a controlled clinical trial against placebo. The compound has been administered orally (450 mg/day, three times daily) to 32 post-stroke patients within six months after the event whilst, concurrently, 27 patients received placebo. The two groups of patients were followed during at least 8 weeks and followed up at the end of week 4 and at the end of week 8 on a symptom scale measuring Activities of Daily Living. The nefiracetam-treated patients showed a moderate or remarkable improvement, whereas no patient in the group treated with placebo showed an improvement. Among the above 59 patients, 19 received nefiracetam and 10 received placebo within three months after stroke. 41.7% of the nefiracetam-treated patients showed a moderate or remarkable improvement whilst no improvement has be noted in the patients who received placebo. The difference was significant (p = 0.035, χ² test). Thus, unlike what the literature seemed to suggest, it has been discovered that nefiracetam has the surprising and unique property of showing a dramatically good activity when given early after stroke.

Further, the action of nefiracetam on the patient who had been treated with nefiracetam early after stroke or later was evaluated. The results are shown in Tables below.

### 1. Global Improvement Rating

| Duration from Stroke | Improvement Rate* | | | | | |
|---|---|---|---|---|---|---|
| < 3 months | 13/19 | 68.4% | | | | |
| < 6 months | 16/32 | 50.0% | | Overall | 32.3% | |
| ≧ 6 months | 26/98 | 26.5% | | | | |
| ≧ 12 months | 18/82 | 22.0% | | | | |

| | | | | | | |
|---|---|---|---|---|---|---|
| *: Percent Patients Remarkably or Moderately Improved | | | | | | |

| Duration from Stroke | Improvement Rate | | x Test |
|---|---|---|---|
| < 6 months | 16/32 | 50.0% | P=0.014 |
| ≧ 6 months | 26/98 | 26.5% | |
| < 12 months | 24/48 | 50.0% | P<0.001 |
| ≧ 12 months | 18/82 | 22.0% | |

### 2. Psychiatric Symptoms

| Duration from Stroke | Improvement Rate* | | | | | |
|---|---|---|---|---|---|---|
| < 3 months | 13/19 | 68.4% | | | | |
| < 6 months | 16/32 | 50.0% | | Overall | 30.8% | |
| ≧ 6 months | 24/98 | 24.5% | | | | |
| ≧ 12 months | 16/82 | 19.5% | | | | |

| | | | | | | |
|---|---|---|---|---|---|---|
| *: Percent Patients Remarkably or Moderately Improved | | | | | | |

| Duration from Stroke | Improvement Rate | | χ² Test |
|---|---|---|---|
| < 6 months | 16/32 | 50.0% | P=0.007 |
| ≧ 6 months | 24/98 | 24.5% | |
| < 12 months | 24/48 | 50.0% | P<0.001 |
| ≧ 12 months | 16/82 | 19.5% | |

### 3. Reduced Spontaneity

| Duration from Stroke | Improvement Rate* | | | | | |
|---|---|---|---|---|---|---|
| < 3 months | 11/18 | 61.1% | | | | |
| < 6 months | 14/30 | 46.7% | | Overall | 23.4% | |
| ≧ 6 months | 15/94 | 16.0% | | | | |
| ≧ 12 months | 8/79 | 10.1% | | | | |

| | | | | | | |
|---|---|---|---|---|---|---|
| *: Percent Patients Remarkably or Moderately Improved | | | | | | |

| Duration from Stroke | Improvement Rate | | χ² Test |
|---|---|---|---|
| < 6 months | 14/30 | 46.7% | P<0.001 |
| ≧ 6 months | 15/94 | 16.0% | |
| < 12 months | 21/45 | 46.7% | P<0.001 |
| ≧ 12 months | 8/79 | 10.1% | |

### 4. Emotional Disturbance

| Duration from Stroke | Improvement Rate* | | | | | |
|---|---|---|---|---|---|---|
| < 3 months | 12/17 | 70.6% | | | | |
| < 6 months | 14/29 | 48.3% | | Overall | 22.6% | |
| ≧ 6 months | 14/95 | 14.7% | | | | |
| ≧ 12 months | 7/81 | 8.6% | | | | |

| | | | | | | |
|---|---|---|---|---|---|---|
| *: Percent Patients Remarkably or Moderately Improved | | | | | | |

| Duration from Stroke | Improvement Rate | | χ² Test |
|---|---|---|---|
| < 6 months | 14/29 | 48.3% | P<0.001 |
| ≧ 6 months | 14/95 | 14.7% | |
| < 12 months | 21/43 | 48.8% | P<0.001 |
| ≧ 12 months | 7/81 | 8.6% | |

### 5. Intellectual Dysfunction

| Duration from Stroke | Improvement Rate* | | | | | |
|---|---|---|---|---|---|---|
| < 3 months | 7/16 | 43.8% | | | | |
| < 6 months | 8/27 | 29.6% | | Overall | 11.9% | |
| ≧ 6 months | 5/82 | 6.1 % | | | | |
| ≧ 12 months | 4/70 | 5.7% | | | | |

| | | | | | | |
|---|---|---|---|---|---|---|
| *: Percent Patients Remarkably or Moderately Improved | | | | | | |

| Duration from Stroke | Improvement Rate | | χ ² Test |
|---|---|---|---|
| < 6 months | 8/27 | 29.6% | P=0.001 |
| ≧ 6 months | 5/82 | 6.1 % | |
| < 12 months | 9/39 | 23.1% | P=0.007 |
| ≧ 12 months | 4/70 | 5.7% | |

### 6. ADL Improvement

| Duration from Stroke | Improvement Rate* | | χ ² Test |
|---|---|---|---|
| < 3 months | 5/12 | 41.7% | P<0.001 |
| ≧ 3 months | 1/81 | 1.2% | |
| < 6months | 5/23 | 21.7% | P<0.001 |
| ≧ 6 months | 1/70 | 1.4% | |
| < 12 months | 5/35 | 14.3% | P=0.017 |
| ≧ 12 months | 1/58 | 1.7% | |

| | | | |
|---|---|---|---|
| *: Percent Patients Remarkably or Moderately Improved | | | |

According to the results of this study, the early treatment with nefiracetam after stroke objectively improves the recovery from stroke, as shown by the fact that, besides psychiatric symptoms such as emotional disturbance and reduced spontaneity, also intellectual dysfunction dramatically improved in a high percent of nefiracetam-treated patients whilst no improvement was noted in the placebo-treated patients. Moreover, nefiracetam surprisingly tends to improve neurological signs and incontinence of urines. Thus nefiracetam, when administered early after the event, appears to be the first drug which is able to induce a recovery from stroke or, at least, to improve the recovery from stroke.

The mechanism of action of nefiracetam and its salt for this indication, which is not bound to the nootropic activity of the drug, is unknown, but it is believed that its surprising effect in improving ADL of a patient after a stroke or in the recovery of, or at least in improving the recovery of, a post-stroke patient, is due to an action against the neurodegeneration and a true brain repair.

Biological in vitro studies carried out on primary cultures of hippocampal and cortical rat embrio neurons showed that nefiracetam, at concentrations of 0.1, 1, 10 and 100 micromoles/1, displays a neurotrophic effect on said neurons by significantly increasing neurite outgrowth. This effect is similar to that induced by basic Fibroblast Growth Factor (bFGF), for which a function as a neurothrophic factor in the brain has been suggested (R.S. Morrison et al., Proceedings of the National Academy of Siences, 1986, 83, 7537-7541; K. Abe et al., 1990, 53, 221-227) and such an effect is surprisingly potentiated by bFGF in hippocampal neurons. This finding strongly suggests that nefiracetam, which is well absorbed and crosses the hematoencephalic barrier, should allow the regeneration of damaged brain neurons in mammals, for example after a stroke, thus favorising brain repair and can be used for combating neurodegeneration in mammals, particularly after a stroke.

This assumption is confirmed by the effect of nefiracetam on spatial learning and retention in rats with cerebral embolism, treated for 9 days with nefiracetam or vehicle, starting within 24 hours of embolization. More particularly, a clear difference between nefiracetam and vehicle-treated animals was observed in the place-learning watermaze task 7 to 9 days after embolization. Moreover, the effect of nefiracetam is maintained even after washout (at day 17 after embolization). This result is predictive of a brain repair effect and shows that the administration of nefiracetam after a stroke provoked by an embolism induces a recovery of the cognition after the stroke.

Thus, it is a third object of the present invention to provide the use of nefiracetam or its salt for the preparation of medicaments for the recovery, or at least for improving the recovery of, a post-stroke patient as defined above. The treatment consists of administering to a patient an effective amount of nefiracetam, said administration being initiated more than 6 months and within 12 months from the stroke. More particularly, the medicament consists of a pharmaceutical composition containing a pharmacologically effective amount of nefiracetam or its salt, as an active ingredient, and a pharmaceutically acceptable carrier. Said effective amount of nefiracetam is advantageously from 50 60 1200 mg, preferably from 100 to 600 mg per unit dose.

A pharmaceutical composition for treating said post-stroke neurodegeneration may comprise a pharmacologically effective amount of nefiracetam or its salt, as an active ingredient, and a pharmaceutically acceptable carrier.

A pharmaceutical composition for the improvement of ADL in said post-stroke patients may comprise a pharmacologically effective amount of nefiracetam or its salt, as an active ingredient, and a pharmaceutically acceptable carrier.

A pharmaceutical composition for the recovery of, or at least for improving the recovery of, said post-stroke patients, may comprise a pharmacologically effective amount of nefiracetam or its salt, as an active ingredient, and a pharmaceutically acceptable carrier.

As set forth above, said compositions are preferably in orally administrable dosage unit form, said dosage unit forms advantageously containing 50-1200 mg, preferably 100-600 mg, of nefiracetam or its salt per unit dose.

According to one particular embodiment, the use of the present invention is for treating post-stroke neurodegeneration in a mammal. This comprises administering to said mammal in need of said treatment an effective amount of nefiracetam or its salt, more particularly a pharmaceutical composition comprising a pharmacological effective amount of nefiracetam or its salt, as an active ingredient, and a pharmaceutically acceptable carrier. Nefiracetam or its salt is administered to treat neurodegeneration as a consequence of a stroke. Preferred mammals to be treated are post-stroke human patients, advantageously post-stroke patients having impaired ADL.

According to a further particular embodiment, the use of the present invention is for improving ADL in a post-stroke patient. This comprises administering to said patient an effective amount of nefiracetam or its salt, said administration being initiated as defined above.

According to a further particular embodiment, the use of the present invention is for recovering, or at least for improving the recovery of, a post-stroke patient. This comprises administering to said patient an effective amount of nefiracetam or its salt, said administration being initiated as defined above.

These uses are carried out by administering nefiracetam or its salt in pharmaceutical compositions as illustrated above.

The oral pharmaceutical compositions in which form nefiracetam or its salt will normally be utilized, are prepared in a manner well known per se in the pharmaceutical art and usually comprise nefiracetam, as an active ingredient, in admixture or otherwise in association with a pharmaceutically acceptable additives. For making those formulations said active ingredient will usually be mixed with, such as, excipients, distintegrator or disintegrating aids, binders, lubricants, coating agents, coloring agents and diluents.

As pharmaceutically acceptable additives for the manufacture of pharmaceutical compositions, for example, excipients such as glucose, lactose, D-mannitol, starch, and crystalline cellulose; disintegrators or disintegrating aids such as carboxymethylcellulose, starch, and carboxymethylcellulose calcium; binders such as hydroxypropylcellulose, hydroxypropylmethylcellulose, polyvinylpyrrolidone, and gelatine; lubricants such as magnesium stearate and talc; and coating agents such as hydroxypropylmethylcellulose, saccharose, polyethylene glycol, and titanium oxide may be used.

The compositions may be administered to the post-stroke patient for example in the form of tablets, capsules, powders, subtilized granules, granules, dragees, solution, syrups or suspensions.

The example of the pharmaceutical composition;

**Tablets**

| | |
|---|---|
| Nefiracetam | 100 mg |
| Lactose | 60 mg |
| Cornstarch | 30 mg |
| Hyroxypropylcellulose | 4.5 mg |
| Low substituted hydroxypropylcellulose | 5 mg |
| Magnesium stearate | 0.5 mg |
| | 200 mg |

### BEST MODE FOR CARRYING OUT INVENTION

The following Example illustrate the invention without, however, limiting it.

### EXAMPLE 1

### Effect of Nefiracetam on Spatial Learning of Rats with Cerebral Embolism

In male Wistar rats weighing 190-220 g a total of 700 microspheres (48 µm in diameter) were injected into the right common carotid artery of each animal, whereby a quasi-immediate embolism occurs. The embolized animals were randomly divided into 2 groups, each of 13 animals with same neurological deficit, designated as "Control" (embolism plus Vehicle), or "Nefiracetam" (embolism plus nefiracetam 10 mg/kg/day). In addition, a group of 13 "Normal (Sham)" non-embolized animals were used. The administration of nefiracetam or of its vehicle started within the same day of embolization and treatment lasted 9 days. Seven days after embolization, the embolized rats were submitted to a watermaze test, said watermaze being adapted from the Morris water task. The time taken to find the platform (latency) was determined. If a rat failed to find the platform within 180 seconds, the trial was terminated and the rat was assigned a score of 180 seconds. The experiment was carried out in two different sessions. In the first session, at the seventh day embolized rats were submitted to the spatial learning test performed three trials per day for three days (from day 7 to day 9). In the second session, one week after the last day of spatial learning test (i.e. on day 17), a retention test was performed whereby each rat was given three consecutive trials to learn and remember the location of the platform. Table 1 shows the average latency (in seconds) to reach the platform in both spatial learning and retention tests. This table shows the clear difference in learning ability between nefiracetam- and vehicle-treated (control) brain-injuried animals when tested at days 7-9 post-embolism. This difference is statistically significant (p < 0.05 compared to control, t-test). This table also shows a clear effect of nefiracetam in the retention test, wherein a remakable difference between the nefiracetam- and vehicle-treated, embolized animals is observed.

**Table 1**

| Latency (Sec.) | | | | | |
|---|---|---|---|---|---|
| Group | n | Spatial learning test | | | Retention test |
| | | Day 7 | Day 8 | Day 9 | Day 17 |
| Normal (Sham) | 13 | 130.9 ± 16.8 | 61.2 ± 15.0 | 22.5 ±5.9 | 31.7 ± 13.1 |
| Control | 13 | 159.7 ± 11.4 | 146.0 ± 13.0 | 127.5 ± 17.0 | 133.4 ± 15.0 |
| Nefiracetam | 13 | 147.6 ± 14.1 | 83.7 ± 17.4 | 93.1 ±16.2 | 72.2 ± 17.0 |

Table 2 summarizes the number of animals which, under the above-described conditions, failed to find the paltform within 180 seconds.

**Table 2**

| Number of rats which failed to find the platform within 180 Sec. | | | | | |
|---|---|---|---|---|---|
| Group | n | Spatial learning test | | | Retention test |
| | | Day 7 | Day 8 | Day 9 | Day 17 |
| Normal (Sham) | 13 | 5 | 1 | 0 | 1 |
| Control | 13 | 10 | 7 | 5 | 7 |
| Nefiracetam | 13 | 8 | 2 | 2 | 2 |

Table 2 shows the clear difference between the nefiracetam-treated animals and the controls.

### INDUSTRIAL APPLICABILITY

N-(2,6-dimethylphenyl)-2-(2-oxo-1-pyrrolidinyl)acetamide or its salt is used for the preparation of medicaments, particularly of pharmaceutical compositions containing it as an active ingredient, for the treatment of neurodegeneration in post-stroke human patients. The early administration of nefiracetam in the pharmaceutical compositions after a stroke allows an improvement of the Acivities of Daily Living and the recovery, or at least in improvement in the recovery of, post-stroke patients

## Claims

1. Use of nefiracetam or a salt thereof for the preparation of a medicament for the treatment of post-stroke neurodegeneration, wherein said treatment is initiated more than 6 months and within 12 months after said stroke.

2. The use of claim 1, wherein said treatment of post-stroke neurodegeneration is for global improvement.

3. The use of claim 1, wherein said treatment of post-stroke neurodegeneration is for improving psychiatric symptoms, reduced spontaneity, emotional disturbance, intellectual dysfunction, or activities of daily living.

4. The use of any one of the preceding claims, wherein said treatment of neurodegeneration is for the recovery, or at least for improving the recovery, of a post-stroke patient.

5. The use of any one of the preceding claims, in which said medicament is in oral unit dose form.

6. The use of any one of the preceding claims, in which said medicament contains from 50 to 1200 mg of nefiracetam.

7. The use of any one of the preceding claims, in which said medicament contains from 100 to 600 mg of nefiracetam per unit dose.

## Patentansprüche

1. Verwendung von Nefiracetam oder eines Salzes davon zur Herstellung eines Medikaments zur Behandlung der Neurodegeneration nach Schlaganfall, wobei mit der Behandlung mehr als 6 Monate und innerhalb von 12 Monaten nach dem Schlaganfall begonnen wird.

2. Verwendung nach Anspruch 1, wobei die Behandlung der Neurodegeneration nach Schlaganfall für eine globale Verbesserung ist.

3. Verwendung nach Anspruch 1, wobei die Behandlung der Neurodegeneration nach Schlaganfall für eine Verbesserung psychiatrischer Symptome, verminderter Spontaneität, emotionaler Verwirrung, intellektueller Störung oder Aktivitäten des täglichen Lebens ist.

4. Verwendung nach einem der vorhergehenden Ansprüche, wobei die Behandlung der Neurodegeneration für eine Genesung oder wenigstens für eine Verbesserung der Genesung eines Patienten nach Schlaganfall ist.

5. Verwendung nach einem der vorhergehenden Ansprüche, wobei das Medikament in Form einer oralen Einheitsdosis vorliegt.

6. Verwendung nach einem der vorhergehenden Ansprüche, wobei das Medikament 50 bis 1200 mg Nefiracetam enthält.

7. Verwendung nach einem der vorhergehenden Ansprüche, wobei das Medikament 100 bis 600 mg Nefiracetam pro Einheitsdosis enthält.

## Revendications

1. Utilisation du néfiracétame ou d'un sel de celui-ci pour la préparation d'un médicament pour le traitement de la neurodégénérescence post-attaque, où ledit traitement est initié plus de 6 mois et dans les 12 mois qui suivent ladite attaque.

2. Utilisation selon la revendication 1 où ledit traitement de la neurodégénérescence post-attaque est destiné à une amélioration globale.

3. Utilisation selon la revendication 1 où ledit traitement de la neurodégénérescence post-attaque est destiné à améliorer les symptômes psychiatriques, la spontanéité réduite, les troubles émotionnels, le dysfonctionnement intellectuel ou les activés de la vie quotidienne.

4. Utilisation selon l'une quelconque des revendications précédentes où ledit traitement de la neurodégénérescence est destiné à la guérison, ou au moins à améliorer la guérison, d'un patient post-attaque.

5. Utilisation selon l'une quelconque des revendications précédentes où ledit médicament est sous forme de dose unitaire orale.

6. Utilisation selon l'une quelconque des revendications précédentes où ledit médicament contient de 50 à 1 200 mg de néfiracétame.

7. Utilisation selon l'une quelconque des revendications précédentes où ledit médicament contient de 100 à 600 mg de néfiracétame par dose unitaire.
